# EUROPEAN PATENT SPECIFICATION

(11) **EP 1 312 364 B1**
(45) Date of publication and mention of the grant of the patent: **22.11.2006**
(21) Application number: 01955609.1
(22) Date of filing: 09.08.2001
(51) Int. Cl.: A61K 31/496, A61P 43/00, A61P 29/00

(54) **GATIFLOXACIN AS CYTOKINE PRODUCTION INHIBITOR**
GATIFLOXACIN ALS INHIBITOR DER CYTOKINPRODUKTION
GATIFLOXACIN COMME INHIBITEUR DE LA PRODUCTION DE CYTOKINES

(30) Priority: 14.08.2000 JP 2000245831
(43) Date of publication of application: 21.05.2003
(73) Proprietor: Senju Pharmaceutical Co., Ltd., Osaka-shi, Osaka 541-0046 (JP); KYORIN PHARMACEUTICAL CO., LTD., Tokyo 101-8311 (JP)
(72) Inventor: TOKUSHIGE, Hideki, Kobe-shi, Hyogo 651-2116 (JP); YOKOGAKI, Shuichi, Kashihara-shi, Nara 634-0007 (JP); NAKA, Hiroaki, Kobe-shi, Hyogo 654-0132 (JP)
(74) Representative: Wright, Robert Gordon McRae
(86) International application number: PCT/JP2001/006839
(87) International publication number: WO 2002/013830

(56) References cited:
- WO-A1-00/18409
- WO-A1-90/01950
- WO-A1-94/20105
- JP-A- 10 130 149
- JP-A- 10 130 240
- US-A- 6 010 711

## Description

### Technical Field

The present invention relates to a cytokine production inhibitor, and more particularly to the use of gatifloxacin (chemical nomenclature: (±)-1-cyclopropyl-6-fluoro-1, 4-dihydro-8-methoxy-7-(3-methyl-1-piperazinyl)-4-oxo-3-quinolinecarboxylic acid), a hydrate thereof, or a salt thereof, for manufacturing a prophylactic or therapeutic medicament for inflammation caused by a cytokine.

### Background Art

Upon infection of bacteria, inflammatory cytokines including tumor necrosis factors (such as TNF-α, etc.) were produced from macrophages and the like stimulated by endotoxins and other factors. The produced cytokines act on neutrophils and vascular endothelial cells to induce inflammatory reactions, which proceed and disappear, involving interactions of many kinds of cells and chemical mediators. Inflammation is one of biophylaxis reactions in the healing process of infectious diseases, but intense or prolonged inflammation increases the physical pain and burden of the patients. In the treatment of infectious diseases, therefore, antimicrobial agents and anti-inflammatory agents are often used in combination.

Today, aphylaxis patients (immunocompromised hosts) can also survive owing to the advanced medicine. However, they easily catch infections by normal flora bacteria or environmental microbes that have been considered as attenuated organisms. Many of these opportunistic infection diseases are hospital-acquired and may often be serious and difficult to treat. Such diseases should be treated with immune system-harmless anti-inflammatory agents and antimicrobial agents. Steroidal anti-inflammatory agents, however, can suppress the immune system and have, if anything, a tendency to induce or enhance infectious diseases and could have various side effects. On the other hand, non-steroidal anti-inflammatory agents can enhance the GABA receptor binding inhibition of new quinolone antibacterial agents and induce spasm. The combination of the non-steroidal anti-inflammatory agents and the new quinolones is therefore contraindicated for systemic administration.

Gatifloxacin which is a new quinolone, is a strong antibacterial compound that can act on gram-positive bacteria and anaerobes as well as gram-negative bacteria (JP 8-9597 B and EP 230295). Conventionally, the new quinolones are reported to have an effect on the production of inflammatory cytokines by macrophages/monocytes or peripheral lymphocytes (Bailly et al., Int. J. Immunopharmac. Vol.12 (1990), pp31-36). WO 00/18409 discloses the use of quinolones, including gatifloxacin, which are TNF-α inhibitors, in the preparation of a pharmaceutical composition for the treatment of spinal disorders such as nerve root injury.

JP 10130149 discloses the use of TNF-α production inhibitors for the treatment of chronic pneumonic diseases. The inhibitors listed in JP 10130149 do not include gatifloxacin.

The anti-inflammatory activity of the new quinolones, however, has never been demonstrated, and practically they have never been used as an anti-inflammatory agent.

### Objects of the Invention

An object of the present invention is to provide gatifloxacin, a hydrate thereof, or a salt thereof as a cytokine production inhibitor, for the prophylaxis or treatment of inflammation caused by a cytokine.

### Disclosure of the Invention

The present inventors have found that gatifloxacin, an antimicrobial agent, inhibits the production of cytokines by macrophages and that it specifically suppresses bacterial infection-induced inflammation, and have completed the present invention.

That is, the present invention provides:
(1) Use of gatifloxacin, a hydrate thereof, or a salt thereof for manufacturing a prophylactic or therapeutic medicament for inflammation caused by a cytokine;
(2) The use according to the above (1), wherein the cytokine is a tumor necrosis factor.

### Brief Description of Drawings

Fig. 1 is a graph showing an inhibitory effect of gatifloxacin on the TNF-α production by lipopolysaccharide (LPS)-stimulated macrophages in Example 1;
Fig. 2 is a graph showing an efficacy of gatifloxacin and the like observed in the cornea in Example 2; and
Fig. 3 is a graph showing an efficacy of gatifloxacin and the like observed in the conjunctiva in Example 2.

### Embodiment for Carrying Out the Invention

Gatifloxacin, a hydrate thereof, or a salt thereof used as an active component of the medicament in the present invention is a known substance as disclosed in JP 8-9597 B and EP 230295 and can be manufactured according to the disclosure thereof. Examples of the hydrate to be used include the 3/2 hydrate as disclosed in JP 8-176143 A and WO96/19472. Examples of the salt can be any pharmaceutically acceptable salt and include mineral acid salts such as hydrochlorides and organic acid salts such as lactic acid salts.

The medicament can be administered to a warm-blooded animal (e.g. a mammal such as human, monkey, dog, cat, pig, rabbit, rat and mouse, and a bird such as chicken, turkey, and pigeon) in need of such inhibition and suppress the production of cytokines. The cytokines play an essential role in the cell-to-cell interaction in vivo, and the production thereof is regulated through the network composed of various cytokines. Upon disturbance of this regulation, abnormal production of the cytokines can occur and lead to various conditions such as allergic diseases, inflammatory diseases, and autoimmune diseases such as chronic rheumatoid arthritis and systemic lupus erythematosus. The cytokines can also be attributable to pathologic neovascularization (such as observed in solid tumor, inflammation, eye diseases such as diabetic retinopathy, psoriasis vulgaris, and atherosclerosis). Thus, the medicament can be used according to the present invention for preventing or treating cytokine-attributable diseases or symptoms as mentioned above, inter alia inflammation caused by a cytokine.

According to the present invention, the medicament for inhibiting cytokine production can be mixed with a pharmaceutically acceptable carrier or vehicle and formed into a pharmaceutical preparation for oral or parenteral administration as desired. The medicament can also be formed into any known dosage form according to known manufacturing techniques such as mixing and dissolving desired components. Examples of the dosage form for oral administration include powders, granules, tablets, capsules, syrups, and liquid preparations. The dosage form of the powder, granule, or tablet can contain any pharmaceutical carrier suited for solid composition preparations, such as a vehicle (e.g. starch, glucose, fructose, and sucrose), a lubricant (e.g. magnesium stearate), a disintegrator (e.g. starch and crystalline cellulose), and a binder (e.g. starch and gum arabic), and can optionally be coated with a coating agent (e.g. gelatin and sucrose). The dosage form of the syrup or liquid preparation can contain any material appropriately selected from a stabilizer (e.g. disodium edetate), a suspending agent (e.g. gum arabic and carmellose), a corrigent (e.g. simple syrup and glucose), and an aromatic. Examples of the parenteral dosage form include injections and suppositories. The injection preparation can contain a solvent (e.g. distilled water for injection), a stabilizer (e.g. disodium edetate), an isotonicity agent (e.g. sodium chloride, glycerol, and mannitol), and a pH adjustor (e.g. hydrochloric acid, citric acid, and sodium hydroxide). The suppository preparation can contain an appropriately selected base (e.g. cacao butter and macrogol).

Examples of the dosage form for topical administration include eye drops, ophthalmic ointments, and nasal drops. The preparation of the eye drop, the ophthalmic ointment, or the nasal drop can contain any known compound appropriately selected from a solvent (e.g. physiological saline and purified water), a stabilizer (e.g. disodium edetate and citric acid), an emulsifier (e.g. polyvinyl pyrrolidone), an emulsion base (e.g. castor oil), a surfactant (e.g. polysorbate 80 and polyoxyethylene hydrogenated castor oil), a preservative (e.g. benzalkonium chloride, parabens, and chlorobutanol), a buffering agent (e.g. boric acid, borax, sodium acetate, citrate buffering agents, and phosphate buffering agents), an isotonicity agent (e.g. sodium chloride, glycerol, and mannitol), a pH adjustor (e.g. hydrochloric acid and sodium hydroxide), an ointment base (e.g. vaseline and lanolin), and a thickening agent (e.g. hydroxypropyl methylcellulose, polyvinyl pyrrolidone, and polyvinyl alcohol).

The medicament for inhibiting cytokine production may be administered in different dosages depending on the symptom or degree of the inflammation, the subject, the age (age in week) and the weight of the subject, the administration route, and so on. For example, the medicament in the form of an internal use is preferably administered to an adult patient with inflammation associated with an infection in internal medicine area or respiratory system, or urinary tract infection at a dose of 100 to 200 mg once to three times per day. The medicament containing gatifloxacin at a concentration of about 0.01w/v% to 5.0w/v%, preferably about 0.05w/v% to 1.0w/v%, in the form of an eye drop is administered to an adult patient with conjunctivitis or the like at a dose of one to several drops once to eight times per day depending on the symptom.

The pharmaceutical preparation may appropriately contain another pharmaceutically active component as far as the object of the present invention can be achieved.

The following Preparation Examples and Examples are provided for the purpose of illustrating the present invention and are not intended to be limiting upon the scope of the invention.

### Preparation Example 1

### Tablet

| | |
|---|---|
| Gatifloxacin | 200 mg |
| Lactose | 80 mg |
| Starch | 17 mg |
| Magnesium Stearate | 3 mg |
| Crystalline Cellulose | 10 mg |

The above components were formed into one tablet by a conventional process. The tablet may be coated with sugar and a film (e.g. hydroxypropyl methylcellulose). Preparation Example 2

### Tablet

| | |
|---|---|
| Gatifloxacin | 400 mg |
| Crystalline Cellulose | 80 mg |
| Low Molecular Weight Hydroxypropyl Cellulose | 30 mg |
| Magnesium Stearate | 12 mg |
| Hydroxypropyl Cellulose | 12 mg |

The above components were formed into one tablet by a conventional process. The tablet may be coated with sugar and a film (e.g. hydroxypropyl methylcellulose). Preparation Example 3

### Granule

| | |
|---|---|
| Gatifloxacin | 75 mg |
| Mannitol | 75 mg |
| Starch | 17 mg |
| Calcium stearate | 3 mg |

The above components were homogeneously mixed and formed into a granule by a conventional process. The granule may be coated with sugar and a film (e.g. hydroxypropyl methylcellulose) as needed.

### Preparation Example 4

### Injection

| | |
|---|---|
| Gatifloxacin | 1000 mg |
| Hydrochloric Acid | 500 mg |
| Sodium Hydroxide | 500 mg |
| Glucose | 3850 mg |
| Distilled Water for Injection Balance | |
| Total | 100 mL |

The gatifloxacin and the hydrochloric acid were dissolved in a small amount of the distilled water for injection, and the glucose was added and dissolved. The sodium hydroxide was then added to adjust the solution to pH 6.0. The distilled water for injection was added to give a total amount of 100 mL of an injection.

### Preparation Example 5

### Injection

| | |
|---|---|
| Gatifloxacin | 1000 mg |
| Lactic Acid | 1750 mg |
| Sodium Hydroxide | 1770 mg |
| Distilled Water for Injection Balance | |
| Total | 100 mL |

The gatifloxacin and the lactic acid were dissolved in a small amount of the distilled water for injection, and the sodium hydroxide was added to adjust the solution to pH 6.0. The distilled water for injection was added to give a total amount of 100 mL of an injection.

### Preparation Example 6

### Eye Drop

| | |
|---|---|
| Gatifloxacin | 0.3 g |
| Sodium Chloride | 0.86 g |
| Hydrochloric Acid | Proper Amount |
| Sodium Hydroxide | Proper Amount |
| Purified Water | Balance |
| Total | 100 mL |

The gatifloxacin and the sodium chloride were added to about 80 mL of the purified water and dissolved. The hydrochloric acid and the sodium hydroxide were added to adjust the solution to pH 6.0. The purified water was added to give a total amount of 100 mL of an eye drop. Preparation Example 7

### Eye Drop

In a similar manner to Preparation Example 6, 0.5 g of the gatifloxacin and 0.83 g of the sodium chloride were used to form an eye drop adjusted to pH 5.5.

### Preparation Example 8

### Eye Drop

| | |
|---|---|
| Gatifloxacin | 0.3 g |
| Sodium Chloride | 0.86 g |
| Benzalkonium Chloride | 0.005 g |
| Disodium Edetate | 0.01 g |
| Hydrochloric Acid | Proper Amount |
| Sodium Hydroxide | Proper Amount |
| Purified Water | Balance |
| Total | 100 mL |

The gatifloxacin, the sodium chloride, the benzalkonium chloride, and the disodium edetate were added to about 80 mL of the purified water and dissolved. The hydrochloric acid and the sodium hydroxide were added to adjust the solution to pH 6.0. The purified water was added to give a total amount of 100 mL of an eye drop. Preparation Example 9

### Eye Drop

In a similar manner to Preparation Example 8, 0.5 g of the gatifloxacin and 0.83 g of the sodium chloride were used to form an eye drop adjusted to pH 5.5.

### Test Example 1

Effect on TNF-alpha Production by Lipopolysaccharide-Stimulated Macrophages

### 1) Test Substances

Gatifloxacin was added to a culture medium at a concentration of 1, 10, or 100 µg/mL.

### 2) Test Method

One mL of 3% thioglycolate culture medium (Sigma) was intraperitoneally administered to C3H/HeNCrj mice (male, 11 weeks old) to induce macrophages. Six days after the administration, the animals were sacrificed, and then 10 mL of sterile RPMI 1640 (GIBCO BRL; 10% FBS-RPMI) containing 100 U/mL penicillin-100 µg/mL streptomycin (GIBCO BRL) and 10v/v% fetal bovine serum (FBS) was intraperitoneally injected. After gentle massage of the abdomen, the 10% FBS-RPMI was collected from the abdomen cavity. After the collected 10% FBS-RPMI was centrifuged, the supernatant was removed and the peritoneal exudate cells including macrophages were harvested. The harvested peritoneal exudate cells were suspended in 10% FBS-RPMI at a concentration of 1 × 10⁶ /mL, and the suspension was plated in each well of a 24-well plate. After incubation for 1.5 hours, non-adherent cells were washed out with 10% FBS-RPMI, and macrophages adhering to the plate were obtained.

Five-hour culture was carried out on each of five groups: a group in which lipopolysaccharide from *Pseudomonas aeruginosa* (LPS: Sigma) was added to the 10% FBS-RPMI at a final concentration of 1 µg/mL; three groups in which the gatifloxacin was added to the 10% FBS-RPMI at a concentration of 1, 10, or 100 µg/mL, respectively, in addition to LPS; and a non-treatment group in which LPS-free 10% FBS-RPMI was used in the culture. Each culture medium was harvested from the culture and centrifuged (12,000 rpm, 4°C, 10 minutes) to remove the cells. Each content of TNF-alpha in the culture supernatant was determined by using an enzyme immunoassay (ELISA).

### 3) Results

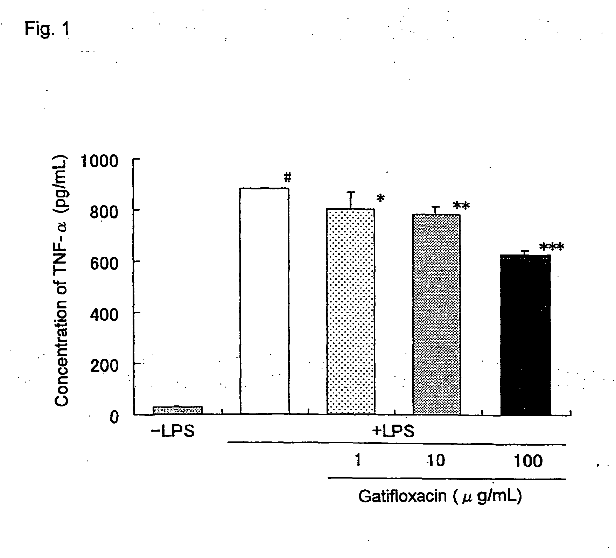

Fig. 1 shows the results of the determined TNF-alpha contents. In the drawing, the ordinate axis represents the TNF-alpha content (pg/mL). Each value is an average ± a standard deviation (n=3). The stimulation by the LPS significantly increased the TNF-alpha content as compared with the non-treatment group(-LPS) (#: p<0.001, Student's t-test: one-sided). The addition of 1 µg/mL or more of the gatifloxacin in combination with the LPS significantly suppressed the increase in the TNF-alpha content (*: p<0.05, **: p<0.01, ***: p<0.001, Williams' test following linear regression analysis: one-sided).

Thus, it was confirmed that the TNF-alpha content of the culture medium in the LPS addition groups was significantly higher than that of the non-treatment group and that the TNF-alpha production in the macrophages was enhanced by the LPS. It was also found that the gatifloxacin dose-dependently inhibited the LPS-stimulated TNF-alpha production and a concentration of 1 µg/mL or more produced a significant inhibitory effect. It has been demonstrated that the gatifloxacin has an inhibitory effect on the TNF-alpha production by the macrophages.

### Test Example 2

### Antiinflammatory Effects on Rabbit Ocular Infection

### 1) Test Substances

The subject substances used for the test include 0.1w/v%, 0.3w/v% and 0.5w/v% gatifloxacin eye drops. The control substances used include a 0.3w/v% ofloxacin eye drop, a 0.5w/v% levofloxacin eye drop and a physiological saline.

### 2) Inoculation of Bacteria

Japanese White Rabbits (male, 1.74 to 2.30 kg in weight) were generally anesthetized, and then 30 µL of 3.1 × 10⁵ CFU/mL methicillin-resistant *Staphylococcus aureus* (MRSA) 0-271 strain suspension was injected into a corneal stroma of each rabbit via a 27 gauge injection needle attached to a 100 µL microsyringe.

### 3) Administration, Observation, and Viable Cell count

Five hours and 24 hours after the bacterial inoculation, 50 µL of each test substance was instillated into the rabbit eye using a micropipet. In the observation carried out 8, 16, 24, 32, and 48 hours after the bacterial inoculation, symptoms such as opacity of cornea, and redness and edema of conjunctiva were observed and scored based on the criteria as shown in Table 1. Each rabbit was sacrificed 52 hours after the bacterial inoculation, and then the eyeball was enucleated and cornea and conjunctiva were cut out. These tissue sections were morcellated in 2 mL of a physiological saline, diluted, and plated onto a Mueller-Hinton agar medium, and then the viable bacterial number was determined by counting the colonies formed after 48 hours.

**Table 1**

| Scoring Criteria of Corneal and Conjunctival Findings in Rabbit Ocular Infection | | | | |
|---|---|---|---|---|
| Items | | | Scoring Criteria | Score |
| Cornea | Corneal opacity * | Degree | Slightly opaque | 1.0 |
| | | | Moderately opaque | 2.0 |
| | | | Iris details obscured, anterior chamber hardly visible | 3.0 |
| | | | Anterior chamber invisible | 4.0 |
| | | Area | One quarter or less but not zero | 0.5 |
| | | | Greater than one quarter but less than one half | 1.0 |
| | | | Greater than one half but less than three quarters | 1.5 |
| | | | Greater than three quarters | 2.0 |
| | Corneal Ulcer | | Crater-like surface irregularities | 1.0 |
| | | | Cornea protruding, surface ulcered | 2.0 |
| | | | Cornea perforated | 3.0 |
| | Inoculation Impression | | Slight impression | 0.5 |
| | | | Overt impression | 1.0 |
| | | | Indistinguishable from corneal opacity | 2.0 |
| Conjun ctiva | Redness of palpebral Conjunctiva | | Slightly reddish mucosa, slightly dilation of vessels in the lid margins | 1.0 |
| | | | Distinctly reddish mucosa, marked dilation of vessels | 2.0 |
| | | | Marked reddish mucosa, unclear running of peripheral vessels | 3.0 |
| | | | Intense hyperemia, diffuse beefy red | 4.0 |
| | Edema of palpebral Conjunctiva | | A tendency of slight edema | 0.5 |
| | | | Any above normal swelling | 1.0 |
| | | | Obvious swelling with partial eversion of the lids | 2.0 |
| | | | Swelling with lids about half closed | 3.0 |
| | | | Severe swelling with lids about half closed to completely closed | 4.0 |
| | Redness of bulbar Conjunctiva | | Slight dilation of corneal limbal vessels | 0.5 |
| | | | More remarkable vasodilation | 1.0 |
| | | | Vessels toward lid markedly dilated or deep red | 2.0 |
| | Condition of nictiating membrane | | Tendencies of vasodilation and edema | 1.0 |
| | | | More prominent vasodilation, with redness of the lid margins | 2.0 |
| | | | Markedly vasodilation, the whole nictitating membrane tinged with red | 3.0 |

| | | | | |
|---|---|---|---|---|
| *: The score of corneal opacity is a calculation of (Degree) × (Area). | | | | |

### 4) Results

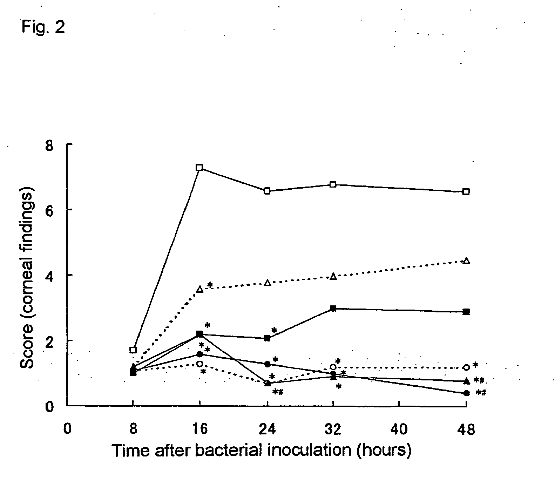

Fig. 2 shows the time course of the corneal findings until 48 hours after the bacterial inoculation. In the drawing, -□- represents the physiological saline eye drop group (n=7), -■- represents the 0.1w/v% gatifloxacin eye drop group (n=7), -▲- represents the 0.3w/v% gatifloxacin eye drop group (n=7), -●- represents the 0.5w/v% gatifloxacin eye drop group (n=8), -Δ- represents the 0.3w/v% ofloxacin eye drop group (n=7), and -o- represents the 0.5w/v% levofloxacin eye drop group (n=9). The ordinate axis represents an average of the total cornea score in each group, and the abscissa axis represents the time after the bacterial inoculation. The mark * means a significant difference between the physiological saline eye drop group and any of the other groups at a risk rate of p<0.05 in a Dunnett's multiple comparison test (one-sided). The mark # means a significant difference between the 0.3w/v% or 0.5w/v% gatifloxacin eye drop group and the 0.3w/v% ofloxacin eye drop group at a risk rate of p<0.05 in the same test. Thus, in the gatifloxacin eye drop groups, the symptoms of the corneal infection were dose-dependently inhibited. The gatifloxacin eye drop group with a concentration of 0.3w/v% or more clearly showed a stronger inhibitory effect than the 0.3w/v% ofloxacin eye drop group. Both of the 0.5w/v% gatifloxacin eye drop group and the 0.5w/v% levofloxacin eye drop group showed a strong inhibitory effect and no difference was found between them.

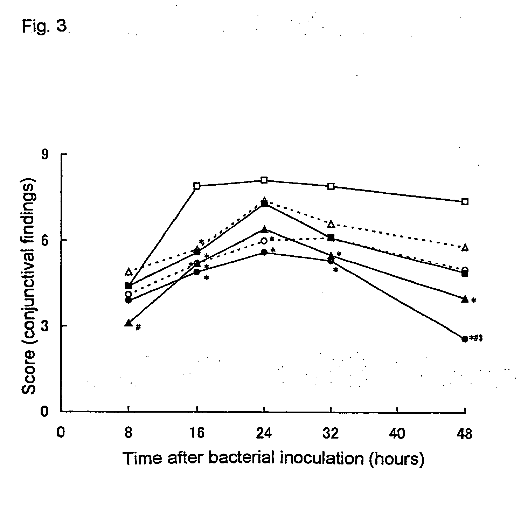

Fig. 3 shows the time course of the conjunctival findings. In the drawing, -□- represents the physiological saline eye drop group (n=7), -■- represents the 0.1w/v% gatifloxacin eye drop group (n=7), -▲- represents the 0.3w/v% gatifloxacin eye drop group (n=7), -●- represents the 0.5w/v% gatifloxacin eye drop group (n=8), -Δ-represents the 0.3w/v% ofloxacin eye drop group (n=7), and -o- represents the 0.5w/v% levofloxacin eye drop group (n=9). The ordinate axis represents an average of the total conjunctiva score in each group, and the abscissa axis represents the time after the bacterial inoculation. The mark * means a significant difference between the physiological saline eye drop group and any of the other groups at a risk rate of p<0.05 in a Dunnett's multiple comparison test (one-sided). The mark # means a significant difference between the 0.3w/v% or 0.5w/v% gatifloxacin eye drop group and the 0.3w/v% ofloxacin eye drop group at a risk rate of p<0.05 in the same test. The mark $ means a significant difference between the 0.5w/v% gatifloxacin eye drop group and the 0.5w/v% levofloxacin eye drop group at a risk rate of p<0.05 in a Student's t-test (one-sided). As shown in Fig. 3, the effect of each substance had a similar tendency to that in the corneal findings until 24 hours after the bacterial inoculation, and after 32 hours, the 0.3w/v% and 0.5w/v% gatifloxacin eye drop groups began to show a stronger inhibitory effect than the ofloxacin or the levofloxacin eye drop group. After 48 hours, the 0.5w/v% gatifloxacin eye drop group showed a significantly stronger inhibitory effect than the 0.5w/v% levofloxacin eye drop group.

Table 2 shows the measured viable bacterial number. In the gatifloxacin eye drop groups, the viable bacterial number in the corneal tissue decreased dose-dependently. In the 0.5w/v% gatifloxacin and the 0.5w/v% levofloxacin eye drop groups, the viable bacterial number greatly decreased similarly. In the 0.3w/v% ofloxacin eye drop group, the decrease effect was relatively weak. On the other hand, no bacterium was detected in the conjunctival tissue in all the groups.

**Table 2**

| Viable Bacterial Number in Corneal and Conjunctival Tissues | | | | |
|---|---|---|---|---|
| Test Substances | | Viable Bacterial Number* | | Number of Samples |
| | | (CFU/Cornea) | (CFU/Conjunctiva) | |
| Physiological Saline | | 2.9 × 10⁶ | ND** | 3 |
| 0.3% Ofloxacin | | 2.0 × 10⁵ | ND | 4 |
| 0.5% Levofloxacin | | 4.4 × 10² | ND | 4 |
| Gatifloxacin | 0.1% | 1.3 × 10⁴ | ND | 4 |
| | 0.3% | 1.1 × 10³ | ND | 4 |
| | 0.5% | 3.9 × 10² | ND | 4 |

| | | | | |
|---|---|---|---|---|
| *: Average, **: Not Detected | | | | |

Based on the results, the following is discussed. The corneal findings were in correlation with the viable bacterial number and therefore the inhibitory effect of the gatifloxacin, levofloxacin, and ofloxacin on the cornea symptoms such as opacity and ulcer would be based on the known bactericidal or bacteriostatic action of these compounds. In the conjunctival tissue, no viable bacterium was detected in all the groups including the physiological saline eye drop group. Therefore, the inflammatory symptoms in the conjunctiva such as redness and edema should secondarily be caused by infiltration of the infection-induced neutrophils and macrophages, and the infiltrating cell-produced inflammatory cytokines and active oxygen. As shown in the results, therefore, the inhibition of the bacterial growth in the infected cornea can secondarily lead to some inhibition of the inflammatory symptoms in the conjunctiva. However, the gatifloxacin significantly showed an excellent inhibitory effect in the conjunctiva with no bacterium detected, though not only the 0.5w/v% gatifloxacin but also the 0.5w/v% levofloxacin similarly showed a bactericidal or bacteriostatic action and a symptom-inhibiting effect in the cornea. This demonstrates that the gatifloxacin has an activity of specifically inhibiting inflammation.

The above-described results show that gatifloxacin has an activity of inhibiting cytokine production as well as the bactericidal or bacteriostatic activity and therefore it would be useful as a prophylactic or therapeutic medicament for inflammation.

## Claims

1. Use of gatifloxacin, a hydrate thereof, or a salt thereof for manufacturing a prophylactic or therapeutic medicament for inflammation caused by a cytokine.

2. The use according to claim 1, wherein the cytokine is a tumor necrosis factor.

## Patentansprüche

1. Verwendung von Gatifloxacin, eines Hydrats davon oder eines Salzes davon zur Herstellung eines prophylaktischen oder therapeutischen Medikaments gegen Entzündung, die durch ein Cytokin verursacht wird.

2. Verwendung gemäß Anspruch 1, wobei das Cytokin ein Tumornekrosefaktor ist.

## Revendications

1. Emploi de gatifloxacine, de l'un de ses hydrates ou de l'un de ses sels en vue de la fabrication d'un médicament prophylactique ou thérapeutique indiqué en cas d'inflammation provoquée par une cytokine.

2. Emploi conforme à la revendication 1, la cytokine étant un facteur de nécrose de tumeur.
